# EUROPEAN PATENT SPECIFICATION

(11) **EP 0 804 558 B1**
(45) Date of publication and mention of the grant of the patent: **23.01.2008**
(21) Application number: 95935875.5
(22) Date of filing: 08.11.1995
(51) Int. Cl.: C12N 9/48, C12P 21/00

(54) **TRIPEPTIDYL AMINOPEPTIDASE**
TRIPEPTIDYL-AMINOPEPTIDASE
TRIPEPTIDYL AMINOPEPTIDASE

(30) Priority: 08.11.1994 DK 128894; 22.12.1994 DK 147094
(43) Date of publication of application: 05.11.1997
(73) Proprietor: Novozymes A/S, 2880 Bagsvaerd (DK)
(72) Inventor: HOLM, Kaj, André, DK-2880 Bagsvaerd (DK); RASMUSSEN, Grethe, DK-2880 Bagsvaerd (DK); HALKIER, Torben, DK-2880 Bagsvaerd (DK); LEHMBECK, Jan, DK-2880 Bagsvaerd (DK)
(74) Representative: Pedersen, Karen Oestergaard
(86) International application number: PCT/DK1995/000446
(87) International publication number: WO 1996/014404

(56) References cited:
- EP-A- 0 440 303
- WO-A-92/16642
- WO-A-95/17512
- CHEMICAL ABSTRACTS, Volume 86, No. 15, 11 April 1977, (Columbus, Ohio, USA), TSYPEROVICH A.S. et al., "System of Aminopeptidases from Aspergillus Flavus", page 166, Abstract No. 102356p; & BIOKHIM. ZH., 1977, 49(1), 101-106.
- CHEMICAL ABSTRACTS, Volume 122, No. 1, 2 January 1995, (Columbus, Ohio, USA), KRIEGER TIMOTHY J. et al., "Purification and Characterization of a Novel Tripeptidyl Aminopeptidase from Streptomyces Lividans 66", page 4118, Abstract No. 4113z; & FEBS LETT., 1994, 352(3), 385-388.
- CHEMICAL ABSTRACTS, Volume 100, No. 4, 23 January 1984, (Columbus, Ohio, USA), BAALOEW ROS MARI et al., "Tripeptidyl Aminopeptidase in the Extralysosomal Fraction of Rat Liver", page 224, Abstract No. 2581x; & J. BIOL. CHEM., 1983, 258(19), 11622-11628.
- CHEMICAL ABSTRACTS, Volume 113, No. 15, 8 October 1990, (Columbus, Ohio, USA), LEES TIMOTHY et al., "Purification and Characterization of Tripeptidyl Aminopeptidase from Human Cerebral Cortex", page 279, Abstract No. 128464y; & BIOCHEM. SOC. TRANS., 1990, 18(4), 667.
- MATTERN I E; ET AL: "Isolation and characterization of mutants of Aspergillus niger deficient in extracellular proteases" MOLECULAR AND GENERAL GENETICS, SPRINGER VERLAG, vol. 234, no. 2, - 1 August 1992 (1992-08-01) pages 332-336, BERLIN, DE
- KRIEGER J T ET AL: "Purification and characterization of a novel tripeptidyl aminopeptidase from Streptomyces lividans 66" FEBS LETTERS, vol. 352, 3 October 1994 (1994-10-03), pages 385-388,

## Description

### FIELD OF THE INVENTION

The present invention relates to a tripeptidyl aminopeptidase (TPAP), a DNA construct encoding the TPAP, a method of producing TPAP and methods of reducing the TPAP production in cells, in which TPAP activity is undesired.

### BACKGROUND OF THE INVENTION

Tripeptidyl aminopeptidases are enzymes capable of cleaving fragments from unsubstituted N-termini of peptides, oligopeptides, or proteins. Tripeptidyl aminopeptidases may be unspecific, i.e. cleaving any tripeptide sequence from the unsubstituted N-terminal end, or specific, i.e. capable of cleaving specific types of tripeptide sequences.

Tripeptidyl aminopeptidases of animal origin have been reported previously. For instance, Doebber et al. (1978), Endocrinology 103: 1794-1804, disclose a tripeptidyl aminopeptidase isolated from bovine pituitary glands, which was shown to cleave tripeptides from the N-terminal end of bovine growth hormone. In Mammalian Proteases Vol. 2, Exopeptidases (AP 1986, eds. J.K. McDonald and A.J. Barrett) tripeptidyl aminopeptidases are disclosed which are isolated from beef pituitary glands, pregnant hog ovaries and hog spleen, respectively.

A bacterial tripeptidyl aminopeptidase isolated from *Streptomyces lividans* 66 is described by Krieger et al., Febs Letters Vol. 352, No. 3, pp 385-388, 1994. Butler et al., Applied and Environmental Microbiology, August 1995, p. 3145-3150 disclose the gene encoding said peptidase and the deduced amino acid sequence. The peptidase is characterized as a serine protease with a pH optimum between 7.5 and 8.5.

It is well-known that the stability of microbially produced products, such as enzymes or other proteins, may vary, inter alia, as a function of the method by which the product is produced and/or the origin or microbial producer of the product. A reduced stability of a microbially produced product may, e.g., be due to a reduced resistance towards heat, light or other external conditions, or may be due to the composition of the product itself. In this connection, the presence of even trace amounts of protease activity in a protein product may result in a significantly reduced stability of said product. Often, however, it is not possible to exactly identify the cause of the varying stability.

### SUMMARY OF THE INVENTION

The present inventors have now surprisingly found that various fungal species produce TPAP and that protein products produced by these organisms may contain minor amounts of TPAP which in some cases have been found to lead to a reduced stability of these products. The present inventors have succeeded in isolating and characterizing said TPAP. It has been established that the TPAP is capable of unspecifically cleaving tripeptides from the unsubstituted N-terminus of protein products, a cleavage which in some instances may be desirable, and in other instances (e.g. when resulting in a reduced stability of a protein product comprising the TPAP) is highly undesirable.

Accordingly, one object of the present invention is to provide a substantially pure tripeptidylpeptidase which may be used for cleaving peptide or protein sequences. Another object is to provide a method of producing protein products essentially free from TPAP, in particular products which are substrates for TPAP and which have a reduced stability in the presence of TPAP.

In a first general aspect the present invention relates to an isolated TPAP of fungal origin. In particular, the TPAP may be obtainable from strains of the fungal species *Aspergillus.* In further aspects the invention relates TPAP identified by amino acid and/or DNA sequence information and/or by enzyme protein charactestics, to DNA constructs encoding the TPAP and to a DNA construct comprising a nucleotide sequence which is complementary to a sufficient part of the DNA sequence encoding TPAP to be able to hybridize to said sequence and thereby abolish the TPAP producing capability of a given host cell.

In a further important aspect the invention relates to a method of reducing the TPAP production from a TPAP producing cell, in which method a DNA sequence encoding TPAP or the TPAP promoter sequence are modified or inactivated so as to result in a reduced TPAP production from said cell.

### DEFINITIONS

In the present context the term "TPAP" is intended to indicate an aminopeptidase which cleaves tripeptides from the N-terminal end of a peptide or protein sequence, for instance from an extended protein sequence, e.g. found in a prohormon or a proenzyme. Expressed in a general manner the TPAP is capable of cleaving the tripeptide XYZ from the unsubstituted N-terminal amino group of a peptide or protein, wherein X, Y, Z represents any amino acid residue (i.e. selected from Ala, Arg, Asn, Asp, Cys, Gln, Glu, Gly, His, Ile, Leu, Lys, Met, Phe, Pro, Ser, Thr, Trp, Tyr, Val). In the TPAP substrate all of X, Y and Z may be different or identical or two of X, Y and Z may be identical. It will be understood that the TPAP of the invention is unspecific as to the amino acid sequence of the tripeptide to be cleaved. In example 7 specific examples of tripeptide products obtained from cleavage of naturally occurring peptides and proteins are given.

The term "obtainable" as used about the origin of DNA sequences constituting part of the DNA construct of the invention is intended to indicate that the DNA sequence in question may be isolated from nucleic acid (DNA or RNA) material of the relevant organism or may be prepared on the basis of such material. For instance, the DNA sequence may be isolated from a genomic or cDNA library prepared from the organism using procedures known in the art, or may be prepared on the basis of such material.

Analogously, when used in connection with the TPAP of the invention the term "obtainable" is intended to indicate that the TPAP may be recovered from the organism in question or may be encoded by a DNA sequence obtainable from said organism and recovered from an organism expressing said DNA sequence.

### DETAILED DISCLOSURE OF THE INVENTION

### The TPAP of the invention

In the course of the research leading to the present invention two different TPAPs have been isolated and characterized - one from a strain of *A. niger,* another from a strain of *A. oryzae.* The two TPAPs are contemplated to be representative examples of a generally novel class of tripeptidyl amino peptidases.

Thus, in one aspect the invention relates to a TPAP which is encoded by a DNA construct comprising
i) at least one of, but preferably two or more of the partial DNA sequences shown in SEQ ID Nos. 1, 2 and 3, or
ii) the partial DNA sequence encoding the N-terminal part of a mature TPAP present in DSM 9570, or
iii) a nucleotide sequence which hybridizes to an oligonucleotide probe prepared on the basis of the DNA sequences shown in SEQ ID No. 1, 2 or 3 or on the basis of any of the amino acid sequences shown in SEQ ID Nos. 4-14, and which encodes a TPAP.

A more detailed explanation of the nucleotide sequence iii) is given futher below in the section entitled "The DNA construct and vector of the invention".

In another aspect the invention relates to a substantially pure TPAP which has one or more of the following characteristics
- a capability of cleaving the substrate Phe-Pro-Ala-pNA,
- a molecular weight of about 65 kDa (determined essentially as described by Laemmli, U.K., 1970, "Cleavage of structural proteins during the assembly of the head of bacteriophage T4"., Nature, 227, p. 680-685)
- a pI in the range of 4-6
- a pH optimum in the range of about 5.0-7.5,
- is immunologically cross-reactive with the purified *A. niger* TPAP or the purified *A. oryzae* TPAP,
- comprises the N-terminal sequence

Ala-Xaa(1)-Asn-Xaa(2)-Ser-His-Cys-Asp-Ser-Ile-Ile-Thr-Pro-Xaa (3)-Cys-Leu-Lys-Xaa(4)-Leu-Tyr-Asn-Ile-Gly-Asp-Tyr-Gln-Ala-Asp-Xaa(5)-Xaa(6) (SEQ ID NO 15), in which any one of Xaa(1), Xaa(2), Xaa(3), Xaa(4), Xaa(5) and Xaa(6) may be different or identical and selected from any of the naturally occurring amino acid residues. Preferably, Xaa(1) is Lys or Gln, Xaa(2) is Ile or Thr, Xaa(3) is Pro or His, Xaa(4) is Glu or Gln, Xaa(5) is Pro or Ala and/or Xaa(6) is Lys or Asn.

Antibodies to be used in determining immunological cross-reactivity may be prepared by use of the purified TPAP. More specifically, antiserum against the enzyme of the invention may be raised by immunizing rabbits (or other rodents) according to the procedure described by N. Axelsen et al. in: A Manual of Quantitative Immunoelectrophoresis, Blackwell Scientific Publications, 1973, Chapter 23, or A. Johnstone and R. Thorpe, Immunochemistry in Practice, Blackwell Scientific Publications, 1982 (more specifically pp. 27-31). Purified immunoglobulins may be obtained from the antisera, for example by salt precipitation [(NH₄)₂SO₄], followed by dialysis and ion exchange chromatography, e.g. on DEAE-Sephadex^{™}. Immunochemical characterization of proteins may be done either by Outcherlony double-diffusion analysis (o. Ouchterlony in: Handbook of Experimental Immunology (D.M. Weir, Ed.), Blackwell Scientific Publications, 1967, pp. 655-706), by crossed immunoelectrophoresis (N. Axelsen et al., *supra,* Chapters 3 and 4), or by rocket immunoelectrophoresis (N. Axelsen et al., *supra,* Chapter 2).

In one embodiment the TPAP of the invention comprises at least one of the partial amino acid sequences shown in SEQ ID Nos. 4-9 and/or has a pH optimum of about 5.0-5.5 and/or a pI of about 5.1 or 5.2.

In another embodiment the TPAP of the invention comprises at least one of the partial amino acid sequences SEQ ID NOs 10-14 and/or has a pH optimum in the range of about 5.5-7.5 and/or a pI of about 4.5.

The TPAP which is encoded by a DNA sequence comprising at least one of the sequences shown in SEQ ID NO 1, 2 and 3 or the one harboured in DSM 9570 or which comprises the peptides shown in SEQ ID NOs 4-9 was isolated from a protein product produced by a strain of *A*. *niger* (cf. Example 1 hereinafter). The TPA comprising the peptides SEQ ID Nos 10-14 was isolated from a strain of *A*. *oryzae,* cf Example 2 hereinafter.

It is presently believed that a TPAP belonging to the generally novel class of tripeptidyl aminopeptides defined herein may be of any origin, including animal or plant origin, but preferably is of microbial, i.e. bacterial or fungal, origin. As far as the present inventors are aware the present disclosure is the first report on TPAP of fungal origin. TPAP of the invention may be purified from strains which are natural TPAP producers, or may more conveniently be produced by means of recombinant DNA techniques as a homologous or heterologous gene product as will be further explained below.

In particular, the TPAP of the invention may be obtainable from a strain of *Aspergillus,* such as a strain of *A*. *oryzae, A. niger, A. japonicus, A. aculeatus, A. nidulans or A. foetidus* or a strain of *Trichoderma,* e.g. *T. viride, T. reesei, T. longibrachiatum* or *T. harzianum,* or a species of *Fusarium,* e.g. *F. oxysporum, F. graminearum* or *F. solani,* or a strain of *Thermomyces,* e.g. *T. lanuginosus* or *T. insolens.*

The TPAP of the invention is preferably provided in an isolated and substantially pure form, e.g. at least 90% pure such as at least 95% pure.

While the presence of TPAP in protein products may be considered undesirable due to the resulting reduced stability of said product, the use of the purified TPAP of the invention for controlled destabilization of protein products may be advantageous. For instance, it is contemplated that the purified TPAP of the invention may be used for deactivation of enzymes after they have exerted their desired effect, and thus, function as a "killer enzyme". Such deactivation is conventionally accomplished by thermoinactivation (or alternatively, the undesired enzyme activity is removed by purification). The use of TPAP for destabilization of thermophilic enzymes may be particularly advantageous.

As an example of this use may be mentioned the deactivation of AMG used for starch liquefaction, which is presently performed by heating the reaction mixture to high temperatures (80-85°C) . This deactivation may, e.g., be obtained by adding TPAP, preferably in a batch proces after AMG has hydrolyzed dextrins to glucose. A thermoinactivation of AMG is then possible by increasing the temperature to only about 66°C for a short time.

Another example is the deactivation of AMG used in the fermentation of beer such as low calorie beer. In the normal beer fermentation procedure AMG is inactivated by pasteurization. The addition of TPAP may reduce the thermostability of the used AMG and thus reduce the temperature for the pasterization. By this treatment the organoleptic characteristics may be improved.

Furthermore, the purified TPAP of the invention may be useful for a number of purposes in which a specific cleavage of tripeptide sequences are desirable. For instance, some proteins or peptides are synthesized in the form of precursors comprising a number of additional amino acid residues on the N-terminal amino acid residue, the presence of which is undesirable for the protein to be used. This may, e.g., be in the processesing of proteins or peptides, which may be synthesized in protected form as precursor proteins.

### The DNA construct and vector of the invention

In accordance with a still further aspect, the invention relates to a DNA construct encoding a TPAP, which comprises
i) at least one of, but preferably two or all of the partial DNA sequences shown in SEQ ID Nos. 1, 2 and 3, or
ii) the partial DNA sequence encoding the N-terminal part of TPAP and present in DSM 9570, or
iii) a nucleotide sequence which hybridizes to an oligonucleotide probe prepared on the basis of any of the DNA sequences shown in SEQ ID No. 1, 2 or 3 or on the basis of any of the amino acid sequences shown in SEQ ID Nos. 4-14, and which encodes a TPAP, or
iv) a nucleotide sequence complementary to the DNA/nucleotide sequence of i), ii) or iii).

The DNA construct of the invention may be used either for the recombinant production of TPAP (DNA/nucleotide sequences i)-iii)) or for reducing the TPAP producing capability of a cell, in which said production is undesired (nucleotide sequence iv)).

The nucleotide sequence iii) may, e.g., be isolated from another or related (e.g. the same) organism known or contemplated to produce TPAP on the basis of any of the partial DNA or partial amino acid sequences shown in SEQ ID Nos. 1-14 or the partial TPAP encoding DNA sequence of DSM 9570, e.g. using the procedures described herein, or constructed on the basis of any of said DNA or amino acid sequences, e.g. by introduction of nucleotide substitutions which do not give rise to another amino acid sequence of the TPAP encoded by the DNA sequence, but which correspond to the codon usage of the host organism intended for production of the TPAP, or by introduction of nucleotide substitutions which do give rise to a different amino acid sequence and therefore, possibly, a different protein structure which might give rise to a TPAP mutant with different properties than the native TPAP, but with the TPAP activity intact. Other examples of possible modifications are insertion of one or more nucleotides into the sequence, addition of one or more nucleotides at either end of the sequence, or deletion of one or more nucleotides at either end or within the sequence.

It will be understood that the DNA sequences shown in SEQ ID Nos. 1-3 and the DNA sequence of DSM 9570 are partial sequences which may be included in and used for isolating an entire TPAP encoding DNA sequence. This may easily be achieved by methods known in the art. In accordance with the present invention, the nucleotide sequence iii) is intended to include said entire DNA sequence.

The hybridization referred to above is intended to indicate that the nucleotide sequence iii) hybridizes to the same probe as the DNA sequence encoding the TPAP (or to said DNA sequence encoding TPAP) under certain specified conditions which are described in detail in the Materials and Methods section hereinafter. Normally, the nucleotide sequence iii) is highly homologous to the DNA sequence shown in SEQ ID No. 1, 2 or 3 or the TPAP encoding DNA sequence of DSM 9570 (when comparing the part of the nucleotide sequence iii) which corresponds to the partial DNA sequence(s) disclosed herein), such as at least 65%, e.g. at least 70% homologous to said DNA sequence, e.g. at least 75%, at least 80%, at least 85%, at least 90% or even at least 95% homologous to any or all of said sequences. Correspondingly, the TPAP of the invention is comtemplated to be at least 65%, e.g. at least 70% homologous to the amino acid sequence encoded by said DNA sequence(s) (as evaluated on the basis of a comparison between the part of the TPAP amino acid sequence corresponding to the part encoded by the DNA sequence in question), e.g. at least 75%, at least 80%, at least 85%, at least 90% or even at least 95% homologous.

The nucleotide sequence iii) may be a DNA or an RNA sequence.

The DNA sequences i)-iii) of the DNA construct of the invention may be prepared by well-known methods. Thus, the relevant DNA sequence may, for instance, be isolated by establishing a cDNA or genomic library from an organism expected to harbour the sequence, e.g. a cell as described above, and screening for positive clones by conventional procedures. Examples of such procedures are hybridization to suitable oligonucleotide probes in accordance with standard techniques (cf. Sambrook et al., Molecular Cloning: A Laboratory Manual, Cold Spring Harbor, New York 1989), and/or selection for clones expressing the relevant activity, and/or selection for clones producing a protein which is reactive with an antibody raised against the relevant enzyme.

A preferred method of isolating a DNA sequence from a cDNA or genomic library is by use of polymerase chain reaction (PCR) using degenerate oligonucleotide probes. For instance, the PCR may be carried out using the techniques described in PCR Protocols 1993, ed. Bruce A. White and The polymerase chain reaction, 1994, ed. Kary B. Mullis.

Alternatively, the DNA sequences i) and ii) may simply be isolated from DSM 9570.

Furthermore, DNA sequences of the DNA construct of the invention, e.g. the nucleotide sequence iv) being complementary to the DNA/nucleotide sequence i), ii) or iii), may be synthesized by established techniques, e.g. based on the principles disclosed by Narang, SA, 1983, Tetrahedron 39:3 and Itakura et al., 1984, Annu. Rev. Biochem. 53:323.

The DNA construct may be of mixed genomic and synthetic, mixed synthetic and cDNA or mixed genomic and cDNA origin prepared by ligating fragments of synthetic, genomic or cDNA origin (as appropriate), the fragments corresponding to various parts of the entire recombinant DNA molecule, in accordance with standard techniques.

It will be understood that a preferred use of the DNA sequence i), ii) or iii) is in the preparation of recombinant TPAP, whereas a preferred use of the DNA sequence iv) is for the reduction of the TPAP producing capability of cells intended for use in the production of protein products which are sensible to TPAP.

Although the DNA sequence iv) may be complementary to the entire TPAP encoding sequence, it is normally sufficient that the DNA sequence iv) is complementary to only part of said sequence. Expressed in a functional manner, the DNA sequence iv) must be complementary to a sufficient length of the DNA sequence encoding the TPAP to allow for hybridization to the TPAP encoding DNA and thus reduction or prohibition of the transcription of said mRNA. Typically, it is sufficient that the DNA sequence iv) comprises a DNA fragment of at least 17 nucleotides such as at least 300 nucleotides.

The vector carrying a DNA construct of the invention, preferably a recombinant expression vector, may be any vector which may conveniently be subjected to recombinant DNA procedures, and the choice of expression vector will often depend on the host cell into which it is to be introduced. Thus, the vector may be an autonomously replicating vector, i.e. a vector which exists as an extrachromosomal entity, the replication of which is independent of chromosomal replication, e.g. a plasmid or a bacteriophage. Alternatively, the vector may be one which, when introduced into a host cell, is integrated into the host cell genome and replicated together with the chromosome(s) into which it has been integrated.

In the DNA construct or the vector, the DNA sequence should be operably connected to a suitable promoter sequence. The promoter may be any DNA sequence which shows transcriptional activity in the host cell of choice and may be derived from genes encoding proteins either homologous or heterologous to the host cell. The promoter may be derived from genes encoding both extracellular and intracellular proteins, such as amylases, glucoamylases, proteases, lipases, cellulases and glycolytic enzymes. Examples of suitable promoters for directing the transcription of the DNA construct of the invention are promoters derived from genes for *A. oryzae* TAKA amylase, *Rhizomucor* miehei aspartic proteinase, *A. niger* glucoamylase, *A. niger* neutral α-amylase, *A. niger* acid stable α-amylase, and *Rhizomucor miehei* lipase. Examples of promoters from genes for glycolytic enzymes are TPI, ADH, and PGK. The promoter may also be a homologous promoter, i.e. a gene native to the host strain being used.

The promoter sequence may be provided with linkers for the purpose of introducing specific restriction sites facilitating ligation of the promoter sequence with the gene of choice or with a selected signal peptide or preregion.

The DNA construct and/or expression vector of the invention may also comprise a suitable terminator operably connected to the DNA sequence encoding the TPAP and/or a polyadenylation sequence. The terminator and polyadenylation sequences may be derived from the same sources as the promoters. Enhancer sequences may also be inserted into the construct.

The DNA construct and/or vector may further comprise a DNA sequence enabling the vector to replicate in the host cell in question. Examples of such sequences are the origins of replication of plasmids pUC19, pACYC177, pUB110, pE194, pAMB1 and pIJ702.

The DNA construct and/or vector may also comprise a selectable marker. Examples of selection markers include the *amdS* or *argB, trpC* or *pyrG* (the latter three markers, e.g. from *A. nidulans* or *A. niger*).

The procedure used to construct the DNA construct of the invention comprise ligating the DNA sequences mentioned above the promoter, the terminator and other elements, respectively, and to insert it into suitable vectors containing the information necessary for replication, are well known to persons skilled in the art (cf., for instance, Sambrook et al. *op. cit.*)*.*

### The cell and a method of producing TPAP of the invention

In one embodiment the cell of the invention either comprising a DNA construct or an expression vector of the invention as defined above is used as a host cell in the recombinant production of TPAP of the invention. In this case the DNA construct or expression vector comprises any of the TPAP encoding DNA sequences i)-iii) or the insert of DSM 9570 defined above. The cell may be transformed with the DNA construct, conveniently by integrating the DNA construct in the host chromosome, although the DNA construct may also exist as an extrachromosomal entity. However, the integration is generally considered to be an advantage as the DNA sequence is more likely to be stably maintained in the cell. Integration of the DNA constructs into the host chromosome may be performed according to conventional methods, e.g. by homologous recombination. Alternatively, the cell may be transformed with an expression vector as described below in connection with the different types of host cells.

The cell of the invention may be a cell of a higher organism such as a mammal or an insect, but is preferably a microbial cell, e.g. a bacterial or a fungal (including yeast) cell.

Examples of suitable bacteria are grampositive bacteria such as *Bacillus subtilis, Bacillus licheniformis, Bacillus lentus, Bacillus brevis: Bacillus stearothermophilus, Bacillus alkalophilus, Bacillus amyloliquefaciens, Bacillus coagulans, Bacillus circulans, Bacillus lautus, Bacillus thuringiensis* or *Streptomyces lividans* or *Streptomyces murinus,* or gramnegative bacteria such as *E.coli.* The transformation of the bacteria may for instance be effected by protoplast transformation or by using competent cells in a manner known *per se.*

The yeast organism may favourably be selected from a species of *Saccharomyces* or *Schizosaccharomyces,* e.g. *Saccharomyces cerevisiae.* The filamentous fungus may advantageously belong to a species of *Aspergillus,* e.g. *Aspergillus oryzae, A. nidulans, A. foetidus, A. aculeatus, A. japonicus* or *A. niger,* a species of *Trichoderma,* e.g. *T. reesei, T. longibrachiatum* or *T. harzianum,* or a species of *Fusarium,* e.g. *F. oxysporum, F. graminearum* or *F. solani.* Fungal cells may be transformed by a process involving protoplast formation and transformation of the protoplasts followed by regeneration of the cell wall in a manner known *per se.*

In a further aspect the invention relates to a method of producing TPAP, which method comprises i) culturing a cell of the invention as defined above in a suitable culture medium under conditions permitting expression of the TPAP, and recovering the TPAP from the culture.

The medium used to cultivate the cells may be any conventional medium suitable for growing the host cell in question. Suitable media are available from commercial suppliers or may be prepared according to published recipes (e.g. in catalogues of the American Type Culture Collection). It is believed that the presence of protein in the medium may result in an increased TPAP production.

The TPAP may be recovered from the medium by conventional procedures including separating the cells from the medium by centrifugation or filtration, if necessary after disruption of the cells, precipitating the proteinaceous components of the supernatant or filtrate by means of a salt, e.g. ammonium sulphate, followed by purification by a variety of chromatographic procedures, e.g. ion exchange chromatography, affinity chromatography, or the like.

### Removal or reduction of TPAP activity

The identification of TPAP as a destabilizing factor in microbially produced protein products may have important consequences for the production of a large number of different protein products. Thus, as it has been demonstrated by the present inventors, even minor amounts of TPAP present in a protein product may result in a reduced thermostability of said product. Accordingly, by the present invention it is possible to construct production strains which have a reduced TPAP-producing capability.

The reduction of TPAP production from a TPAP producing cell may conveniently be accomplished by modification or inactivation of a DNA sequence present in said cell and necessary for expression of TPAP so as to result in a reduced TPAP production from said cell.

The DNA sequence to be modified may, e.g., be a DNA sequence encoding TPAP or a part thereof essential for exhibiting TPAP activity or may be a regulatory sequence required for the expression of TPAP from a TPAP encoding DNA sequence.

As an example the regulatory sequence may be a promoter sequence or a functional part thereof, i.e. a part which is sufficient for effecting expression of TPAP.

The modification or inactivation of the DNA sequence may be performed by subjecting the TPAP producing cell to mutagenesis and selecting for cells for which the TPAP producing capability has been reduced. The mutagenesis, which may be specific or random, may, e.g., be performed by use of a suitable physical or chemical mutagenizing agent, by use of a suitable oligonucleotide, or by subjecting the DNA sequence to PCR generated mutagenesis. Furthermore, the mutagenesis may be performed by use of any combination of these mutagenizing agents.

Examples of a physical or chemical mutagenizing agent suitable for the present purpose includes ultraviolet (UV) irradiation, hydroxylamine, N-methyl-N'-nitro-N-nitrosoguanidine (MNNG), O-methyl hydroxylamine, nitrous acid, ethyl methane sulphonate (EMS), sodium bisulphite, formic acid, and nucleotide analogues.

When such agents are used the mutagenesis is typically performed by incubating the cell to be mutagenized in the presence of the mutagenizing agent of choice under suitable conditions for the mutagenesis to take place, and selecting for mutated cells having a reduced TPAP production.

When the modification or inactivation is accomplished by introduction, substitution or removal of one or more nucleotides in the TPAP encoding sequence or a regulatory element required for the transcription or translation thereof, nucleotides may, e.g., be inserted or removed so as to result in the introduction of a stop codon, the removal of the start codon or a change of the open reading frame. The modification or inactivation of the TPAP encoding sequence or a regulatory element may be accomplished by site-directed mutagenesis or PCR generated mutagenesis in accordance with methods known in the art. Although in principle, the modification may be performed *in vivo,* i.e. directly on the cell carrying the TPAP gene to be modified, it is presently preferred to conduct the modification *in vitro* as exemplified below.

An example of a convenient way to inactivate or reduce the TPAP production of a host cell of choice is based on the principles of gene replacement or gene interruption. For instance, the gene interruption method involves the use of a DNA sequence corresponding to the endogenous gene or gene fragment which it is desired to destroy. Said DNA sequence is *in vitro* mutated to a defective gene and transformed into the host cell. By homologous recombination the defective gene replaces the endogenous gene or gene fragment. It may be desirable that the defective gene or gene fragment encodes a marker which may be used for selection of transformants in which the TPAP gene has been modified or destroyed.

Alternatively, the modification or inactivation of the DNA sequence may be performed by use of established anti-sense techniques using a nucleotide sequence complementary to the TPAP encoding sequence, e.g. the nucleotide sequence iv) described above. More specifically, the TPAP production from a TPAP producing cell may be reduced or eliminated by introducing a nucleotide sequence complementary to the TPAP encoding sequence and thus capable of hybridizing to TPAP mRNA produced in the cell into the cell in such a manner that the nucleotide sequence may be transcribed in the cell under conditions allowing the nucleotide sequence to hybridize to the TPAP mRNA and thus reduce the amount of TPAP translated from said mRNA or eliminate any such translation.

The TPAP-deficient mutants so created are particularly useful in the expression of heterologous proteins. In the present context the term "heterologous proteins" is intended to mean a protein which is not native to the host cell, a native protein in which modifications have been made to alter the native sequence, or a native protein whose expression is quantitatively altered as a result of a manipulation of the host cell by recombinant DNA techniques.

It is preferred that the TPAP producing cell to be modified in accordance with the present invention is a strain which is suitable for the production of desired protein products, either homologous or heterologous to the cell. For instance, cells of the fungal genera *Aspergillus, Trichoderma* and *Fusarium* are examples of prefered production cells. Accordingly, the cell to be modified according to the present invention is preferable a cell of an *Aspergillus* sp., in particular a cell of *A. niger, A. oryzae, A. japonicus, A. foetidus* or *A. nidulans* or a cell of a *Trichoderma* sp., e.g. *T. reesei, T. longibrachiatum* or *T. harzianum,* or a cell of a *Fusarium* sp., e.g. *F. oxysporum, F. graminearum* or *F*. *solani.*

In a specific embodiment of the invention the cell to be modified is a cell of *A*. *niger* or *A*. *oryzae* which is used for the production of enzymes such as AMG.

In a further aspect the invention relates to a method of preparing a product essentially free from TPAP activity, which method comprises transforming a host cell as described above having a reduced or no TPAP producing capability with a DNA sequence encoding the product, culturing the transformed cell under suitable conditions for expression of the product, and recovering the product from the culture.

In an alternativ aspect the invention relates to a method of preparing a product essentially free from TPAP activity, which product is encoded by a DNA sequence present in a TPAP expressing cell, which method comprises modifying or inactivation a DNA sequence present in said cell and necessary for expression of TPAP as described above, and subsequently culturing the cell under suitable conditions for expression of the product, and recovering the product from the culture.

In a still further aspect the invention relates to a method of preparing a product essentially free from TPAP by fermentation of a TPAP-producing cell which also produces the product, which method comprises adding an effective amount of an inhibitor capable of inhibiting TPAP activity to the fermentation broth either during or after the fermentation has been completed, recovering the product of interest from the fermentation broth, and optionally subjecting the recovered product to further purification. This method is further illustrated in the examples below.

In a still further alternative aspect the invention relates to a method of preparing a product essentially free from TPAP activity, which product is encoded by a DNA sequence present in a TPAP expressing cell, which method comprises cultivating the TPAP expressing cell encoding the product under conditions permitting the expression of the product, subjecting the resulting culture broth to a combined pH and temperature treatment so as to reduce the TPAP activity substantially, and recovering the product from the culture broth. Alternatively, the combined pH and temperature treatment may be performed on an enzyme preparation recovered from the culture broth. The combined pH and temperature treatment may optionally be used in combination with a treatment with a TPAP inhibitor.

In accordance with this aspect of the invention it is possible to remove at least 60% of the TPAP activity, such as at least 75% of the activity, more preferably at least 85% of the activity, still more preferably at least 95% of the activity, and most preferably essentially at least 99% of the TPAP activity. It is contemplated that a complete removal of TPAP activity may be obtained by use of this method.

The combined pH and temperature treatment is preferably carried out at a pH in the range of 6.5-7 and a temperature in the range of 25-40°C for a sufficient period of time for obtaining the desired effect. Typically, 0.5-1 hour is sufficient for obtaining the desired effect.

The methods used for cultivation and purification of the product of interest may be performed by methods known in the art, e.g. as described herein before.

The methods of the invention for producing an essentially TPAP-free product is of particular interest in the production of eukaryotic proteins, in particular fungal proteins such as enzymes. The enzyme product may, e.g., be selected from an amylolytic enzyme, a lipolytic enzyme, a proteolytic enzyme, a cellulytic enzyme, an oxidoreductase or a plant cell-wall degrading enzyme. Examples of such enzymes include AMG, amylase, lipase, cutinase, esterase, cellulase, hemicellulase, protease, peroxidase, laccase, phenoloxidase, catalase, glucose oxidase, phytase, lyase, pectinase, glucosidase, mannosidase, isomerase, invertase, trasferase, ribonuclease, galactosidase, transglutaminase and chitinase. The TPAP-deficient cells may also be used to express heterologous proteins of pharmaceutical interest such as hormones, growth factors, receptors, and the like.

It will be understood that the term "eukaryotic proteins" is intended to include not only native proteins, but also those proteins (e.g. enzymes) which have been modified by amino acid substitutions, deletions, additions, or other modifications which may be made to enhance activity, thermostability, pH tolerance and the like.

In a further aspect the invention relates to a protein product essentially free from TPAP activity which is produced by the method of the invention.

### EXAMPLE 1

PURIFICATION AND CHARACTERIZATION OF *A. niger* TPAP

### Materials and methods

**Phe-Pro-Ala-pNA substrate** (available from Bachem, Switzerland).

### Protease inhibitors

Ala-Ala-Phe-chloromethylketone
Benzyloxycarbonyl-Ala-Pro-Phe-chloromethylketone
Benzyloxycarbonyl-Gly-Gly-Phe-chloromethyl-ketone.

### Purification of TPAP

TPAP was purified from a commercial *A*. *niger* AMG preparation (available from Novo Nordisk A/S, Denmark). A sample of 300 ml formulated AMG product was repeatedly diluted and concentrated at 4°C in a Filtron^{®} concentrator equipped with a 3 kDa cutoff membrane until the conductivity was less than 1.5 mS/cm. All other purification steps were carried out at ambient temperature.

### Cation exchange chromatography employing a NaCl gradient

The concentrate (600 ml) was adjusted to pH 4.0 and filtrated before application to a 200 ml (2.6 x 37 cm) S-Sepharose column equilibrated with 20 mM sodium acetate, pH 4.0. A flow of 10 ml/min was used. The TPAP was eluted with a linear NaCl gradient from 0 to 0.2 M in 10 column volumes. One pool containing the largest part of the peptidase activity was made. A buffer change to 20 mM sodium acetate, pH 5.5 was made in an Amicon cell equipped with a Diaflo membrane with a cutoff of 10 kDa.

### Anion exhange chromatography

The pool from the S-Sepharose column was further purified on a HiLoad Q-Sepharose HP column (50 ml, 2.6 x 10 cm) equilibrated with 20 mM sodium acetate, pH 5.5. Elution of TPAP was performed with a linear NaCl gradient from 0 to 0.5 M in 15 column volumes. The protein was applied with a flow of 8.0 ml/min and eluted with 5.0 ml/min. A pool containing the largest part of TPAP was made. A buffer change to 20 mM sodium acetate, pH 4.0 was made in an Amicon cell as described above.

### Cation exchange chromatography employing a pH gradient

The pool from the HiLoad Q-Sepharose HP column was finally purified on a Mono S column (5/5) equilibrated with 20 mM sodium acetate, pH 4.0. A gradient from pH 4.0 to pH 6.0 was made in 30 column volumes using 20 mM sodium acetate, pH 4.0 and 20 mM sodium acetate, pH 6.0. The flow was 1.0 ml/min. Two isoenzymes of TPAP eluted at pH 5.1 and 5.2, respectively.

### Purification of AMG

AMG G1 has been purified from a commercial AMG preparation (Novo Nordisk A/S) by anion exchange chromatography using Q-Sepharose. A 50 ml column was equilibrated with 20 mM sodium acetate, pH 5.5 and the G1 form eluted with a linear NaCl gradient from 0 to 0.6 M NaCl in 8 column volumes. The flow was 8.0 ml/min.

AMG_{trunc} was purified after TPAP treatment of AMG G1 on a Mono Q column equilibrated with 20 mM sodium acetate, pH 4.3. A linear NaCl gradient from 0 to 1.0 M in 30 column volumes was used for elution. The flow was 1.0 ml/min.

### Destabilization assay

Aliquots of purified AMG G1 were incubated with different mixtures of TPAP and buffer in 0.1 M sodium acetate, pH 4.3 for several weeks at 37°C. The final volume was either 1 or 2 ml with a concentration of AMG G1 of 10 AGU/ml. 100 µl of the incubation mixture were withdrawn and diluted to 2 AGU/ml with 0.1 M sodium acetate, pH 4.3. A heat treatment at 65°C for 30 min was carried out on the diluted sample. After cooling the samples to ambient temperature, the activity of untreated and heat treated samples were measured in microtiter plates using the chromogenic substrate p-nitrophenyl-α-D-glycopyranoside (pNPG) (Merck, Art. 6792). 50 µl 3 mM pNPG in 0.1 M sodium acetate, pH 4.3 was incubated with 25 µl sample for 30 min at ambient temperature. The reaction was stopped by addition of 75 µl 0.1 M sodium tetraborate. The absorbance at 405 nm was measured in a UV-max kinetic microplate reader. T₃₀ was calculated as the percentage of activity retained after heat - treatment. All measurements were made in duplicate.

### TPAP assay

The assay was performed either in a microtiter plate reader or in a spectrophotometer using a substrate concentration of 0.2 mM Phe-Pro-Ala-pNA in 0.1 M sodium acetate buffer, pH 4.3. A 5 mM stock solution of Phe-Pro-Ala-pNA was made in DMSO and diluted before use. The reaction was followed for 4 min at 405 nm either in a UV-max kinetic microplate reader or a spectrophotometer and the initial rate of cleavage calculated.

### Inhibition of TPAP

Aliquots containing 4 µg TPAP were incubated with a number of protease inhibitors: 1 mM Ala-Ala-Phe-chloromethylketone, 1 mM benzyloxycarbonyl-Ala-Pro-Phe-chloromethylketone and 1 mM benzyloxycarbonyl-Gly-Gly-Phe-chloromethyl-ketone. Following 30 min incubation the residual activity was determined.

### Storage stability of filtrated fermentation broths

The culture broths were centrifuged and sterile filtrated through a 0.22 µm filter. The broths were made 0.1% with respect to potassium sorbate and sodium benzoate and pH was adjusted to 4.3. Aliquots of 2 ml were either added 200 µl of 10 mM Ala-Ala-Phe-chloromethylketone or 200 µl of 0.1 M sodium acetate, pH 4.3. Samples were withdrawn and diluted to 2 AGU/ml before the T₃₀ determination as described above.

### Determination of AMG activity (AGU)

AMG activity was determined as described By K.A. Holm, 1980, Anal. Chem. Acta., 117, pp 359-362. In brief, the method is based on hydrolysis of maltose by AMG under formation of alpha-D-glucose. After a short, continuous dialysing procedure the concentration of glucose is determined by a glucose dehydrogenase (GlucDH) reaction (performed at pH 7.6). Standard conditions for the automated Auto-Analyzer method are:
substrate: maltose 28 mM
Incubation buffer: acetate 0.1M, pH 4.3
Incubation temperature: 37°C
Incubation time: 5 min.

The enzyme-working area of the method is 0.5-4 AGU/ml.

### Results

TPAP was purified to homogeniety from a commercial AMG preparation (Novo Nordisk A/S) according to the purification scheme shown in Table 1. The elution profile from the final cation exchange column revealed the presence of two isoenzymes of TPAP (TPAP-I and TPAP-II) with pI 5.1 and 5.2, respectively. Both isoforms were pure as judged by SDS-PAGE and N-terminal sequencing, but the specific activities of the enzymes differed (cf. Table 1). TPAP-II had 20% higher specific activity than TPAP-I.

A deamidation of one or several Asn- or Gln-residues either in the fermentation broth or during purification can explain the small difference in pI between the two isoforms of TPAP.

**Table 1. Purification of TPAP from a commercial AMG preparation**

| | Pools | Volume | Total protein | Total activity* µmol/min | Specific activity | Yield | Purification fold |
|---|---|---|---|---|---|---|---|
| | | ml | mg^{#} | | µmol/min/ mg^{#} | % | |
| UF-concentrate | | 600 | 117,000 | 1,900 | 0.016 | 100 | - |
| S-sepharose | | 250 | 410 | 1,100 | 2.7 | 58 | 170 |
| HPQ-sepharose | | 45 | 27 | 390 | 14 | 20 | 880 |
| Mono S | TPAP-I | 8.5 | 6.0 | 132 | 22 | 7 | 1380 |
| | TPAP-II | 8 | 4.9 | 132 | 27 | 7 | 1680 |

| | | | | | | | |
|---|---|---|---|---|---|---|---|
| #: based on the assumption that 1 mg/ml gives A₂₈₀ = 1 *: measured as release of pNA in the TPAP assay | | | | | | | |

### pH optimum

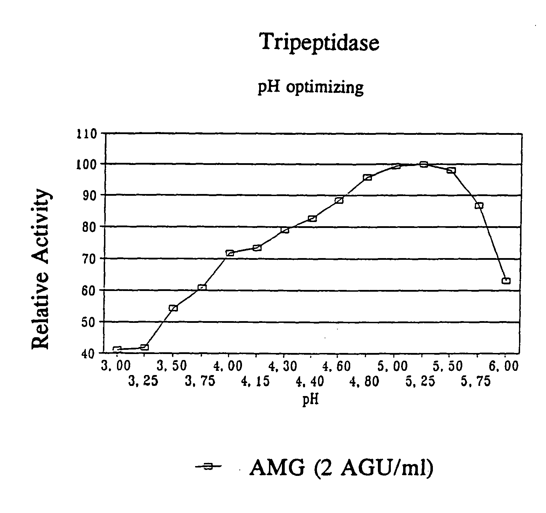

The pH optimum of TPAP was determined using the same procedure as in the TPAP assay described above. 0.1 M acetate buffer was used to regulate the pH. The resulting pH optimum curve is shown in Fig. 1. It is seen that the TPAP functions optimally at a pH in the range of 5.0-5.5, in particular 5.25.

### Determination of temperature optimum for TPAP

The temperature/activity relationship of TPAP was determined in 0.1 M sodium acetate buffer, pH 5.5 using the TPAP assay described above. As can be seen from the table TPAP has maximal activity in the temperature range 45-55 °C.

**Table 2**

| | | | | | | | | | |
|---|---|---|---|---|---|---|---|---|---|
| Temperature | 25 | 30 | 35 | 40 | 45 | 50 | 55 | 60 | 65 |
| (°C) | | | | | | | | | |
| Rel. act. | 42 | 52 | 63 | 78 | 100 | 99 | 98 | 51 | 8 |
| (%) | | | | | | | | | |

### Mass spectrometry

Matrix assisted laser desorption ionisation time-of-flight mass spectrometry of TPAP purified from *A. niger* gave a broad signal indicating that the TPAP is glycosylated. The average mass was found to be 54.5 kDa.

### EXAMPLE 2

PURIFICATION AND CHARACTERIZATION OF *A*. *oryzae* TPAP

### Materials & Methods

As a starting material for the purification, the supernatant of an *A*. *oryzae* IFO 4177 fermentation fermented at pH 5 in a soy containing medium was used. After fermentation, the culture broth was centrifuged to remove the majority of cells.

### Purification

Approx. 4L supernatant was germ filtered on a Seitz EKS plate. The EKS-filtrate was ultrafiltrated on a 3k cut-off Filtron cassette (Minisette) to minimal volume. Ultrafiltrate = 240 ml. 170 ml of the ultrafiltrate was precipitated with solid ammonium sulphate (AMS) to give an AMS saturation of approx. 90%. After stirring for at least 30 min., the AMS precipitate was recovered by centrifugation in a Sorvall RC3B centrifuge (4500 rpm, 15 min., room temp). 100 ml deionized water was added to the AMS precipitate to dissolve the protein and 1% (w/v) FGV120 activated charcoal was added to remove colour. After stirring for approx. 1 hour the suspension was filtered on a Seitz EK1 plate to remove charcoal (and colour). The EK1-filtrate was dialysed against 1) 100 mM H₃BO₃, 10 mM dimethyl glutaric acid, 2 mM CaCl₂, pH 5 and 2) deionized water. After an EK1-filtration the dialysate (260 ml) was freezed in aliquots.

A 120 ml aliquot of the dialysate was thawed and applied to a 1.4L G25 Sephadex column equilibrated in 20 mM CH₃COOH/NaOH, pH 5.0. To remove colour and very acidic proteins, the G25-filtrate was applied to a 40 ml Q-sepharose FF column equilibrated in the same buffer (CH₃COOH/NaOH, pH 5.0). After washing the column, bound protein was eluted with a linear NaCl gradient (0 → 200mM). Fractions from the column were analysed for TPAP activity. Most of the TPAP activity was seen in the run-through (70%), whereas most of the protein was bound to the column.

The run-through from the Q-sepharose column was applied to a 50 ml S-sepharose HP column equilibrated in 20 mM CH₃COOH/NaOH, pH 5.0. After washing the column, bound protein was eluted with a linear NaCl gradient (0 → 200mM). Fractions from the column were analysed for TPAP activity. Most of the TPAP activity was again seen in the run-through (75%). The rest of the TPAP activity was in the start of the NaCl gradient. The run-through + fractions 1-11 were pooled and dialysed against 50 mM H₃BO₃, 5 mM dimethyl glutaric acid, 1 mM CaCl₂, pH 6.0.

After adjusting the pH of the dialysed pool to pH 7.0 the enzyme was applied to a 23 ml SOURCE Q column equilibrated in 50 mM H₃BO₃, 5 mM dimethyl glutaric acid, 1 mM CaCl₂, pH 7.0. After washing the column, bound protein was eluted with a linear NaCl gradient (0 → 500 mM). Fractions from the column were analysed for TPAP activity. All the TPAP activity was seen in the run-through (95%). The reason for this has to be, that the column had been overloaded with protein. The run-through was dialysed against 20 mM CH₃COOH/NaOH, pH 4.0.

The dialysed enzyme was applied to a 50 ml S-sepharose HP column equilibrated in 20 mM CH₃COOH/NaOH, pH 4.0. After washing the column, bound protein was eluted with a NaCl gradient (0 → 200 mM). Fractions from the column were analysed for TPAP activity. The TPAP activity eluted with 100 mM NaCl. The TPAP activity was pooled and dialysed against 20 mM CH₃COOH/NaOH, pH 4.0. To get a better resolution than what the S-sepharose column could give, the dialysed pool was applied to an 8 ml SOURCE S column equilibrated in the same buffer (20 mM CH₃COOH/-NaOH, pH 4.0). After washing the column, bound protein was eluted with a NaCl gradient (0 → 200 mM). Fractions from the column were analysed for TPAP activity. The TPAP activity eluted with 60 mM NaCl. The TPAP activity was pooled and dialysed against 20 mM CH₃COOH/NaOH, pH 5.5.

The dialysed enzyme was applied to a 23 ml SOURCE Q column equilibrated in 20 mM CH₃COOH/NaOH, pH 5.5. After washing the column, bound protein was eluted with a NaCl gradient (0 → 200 mM). The TPAP activity eluted with 30 mM NaCl. Fractions from the column were analysed by SDS-PAGE and for TPAP activity. The SDS-PAGE gel showed that the fractions containing TPAP activity contained two major bands: a 65 kDa band (TPAP) and a 30 kDa band. The TPAP band was diffuse whereas the 30 kDa band was sharp, indicating that TPAP is glycosylated whereas the 30 kDa band is not.

The TPAP containing fractions were concentrated to 1 ml by ultrafiltration and applied to a 150 ml Sephacryl S-100 column equilibrated in 20 mM Tris-acetate, 100 mM NaCl, pH 5.5. With a 1.0 ml/min flow rate the two bands were separated. The TPAP activity containing fractions were pooled as *A.oryzae* TPAP.

### Mass spectrometry

Matrix assisted laser desorption ionisation time-of-flight mass spectrometry of TPAP purified from *A*. *oryzae* gave a broad signal indicating that the TPAP is glycosylated. The average mass was found to be 55.0 kDa.

### pH profile of A. oryzae TPAP

The pH dependency of the activity of TPAP from *A*. *oryzae* was investigated using the chromogenic substrate Phe-Pro-Ala-pNA. To 50 µl of enzyme 150 µl Britton-Robinson buffer at the indicated pH were added before incubation with 50 µl Phe-Pro-Ala-pNA at a final substrate concentration of 0.2 mM. The assays were performed in an UV-max kinetic microtiter plate reader and the reaction followed for 3.5 min at 405 nm. The relative activity (RA) is given in the table 3 below.

**Table 3**

| | | | | | | | | | | | |
|---|---|---|---|---|---|---|---|---|---|---|---|
| pH | 4.0 | 4.5 | 5.0 | 5.5 | 6.0 | 6.5 | 7.0 | 7.5 | 8.0 | 8.5 | 9.0 |
| RA | 13 | 11 | 14 | 26 | 61 | 100 | 74 | 29 | 1 | 0 | 0 |

### EXAMPLE 3

### Peptide sequences of A. niger TPAP

N-terminal amino acid sequencing of intact *A*. *niger* TPAP forms I and II (Example 1) as well as of peptides derived from TPAP form I was performed in an Applied Biosystems 473A sequencer operated according to the manufacturers instructions.

The N-terminal amino acid sequences of intact TPAP forms I and II were determined for 30 residues. The two sequences were identical and found to be:
Ala-Gln-Asn-Thr-Ser-His-Cys-Asp-Ser-Ile-Ile-Thr-Pro-His-Cys-Leu-Lys-Gln-Leu-Tyr-Asn-Ile-Gly-Asp-Tyr-Gln-Ala-Asp-Pro-Lys-(SEQ ID No. 4)

The sequence did not show any homology to other proteins.

Following denaturation, reduction and S-carboxymethylation, peptides were derived from TPAP form I by proteolytic cleavage using the lysyl-specific protease from *Achromobacter.* The resulting peptides were fractionated and repurified using revesed phase HPLC. The purity and mass of the peptides were evaluated using matrix assisted laser desorption ionisation time-of-flight mass spectrometry using a VG Analytical TofSpec operated according to the manufacturers recommandations. The following 7 peptides were sequenced.
*Peptide 1*:
   Ala-Gln-Asn-Thr-Ser-His-Cys-Asp-Ser-Ile-Ile-Thr-Pro-His-Cys-Leu-Lys
   Asn3 is glycosylated as shown by mass spectrometry and amino acid sequencing. Peptide 1 is identical to amino acid residues 1-17 of intact TPAP and thus of the sequence shown in SEQ ID No. 4.
*Peptide 2:*
   Gln-Leu-Tyr-Asn-Ile-Gly-Asp-Tyr-Gln-Ala-Asp-Pro-Lys
   Peptide 2 is identical to amino acid residues 18-30 of intact TPAP (and thus of the amino acid sequence shown in SEQ ID No. 4). Peptide 2 is recovered in two forms as revealed by mass spectrometry and amino acid sequencing. One form with an N-terminal Gln residue and one form with this Gln residue converted to a pyroglutamate residue.
*Peptide 3:*
   Thr-Ser-Pro-Glu-Gln-Ala-Val-Ser-Phe-Ser-Ser-Gly-Gly-Phe-Ser-Asp-Leu-Trp-Pro-Arg-Pro-Ser-Tyr-Gln-His- (SEQ ID No. 5)
*Peptide 4:*
   Phe-Ser-Gly-Leu-Phe-Asn-Ala-Ser-Gly-Arg-Ala-Phe-Pro-Asp-Val-Ser-Ala-Gln-Gly-Val-Asn-Tyr-Ala-Val-Tyr-Asp-Lys (SEQ ID No. 6)
   Asn6 is glycosylated as shown by mass spectrometry and amino acid sequencing.
*Peptide 5:*
   Ile-Gly-Phe-Ala-Ser-Tyr-Leu-Gln-Glu-Tyr-Ala-Arg-Tyr-Ala-Asx-Leu-Glu-Arg-Phe-Glu-Gln-His-Leu- (SEQ ID No. 7)
   It was not possible to discriminate whether Asx15 was an Asp or Asn residue.
*Peptide 6:*
   Xaa-Leu-Asx-Leu-Gln-Tyr-Ile-Leu-Gly-Val-Ser-Ala-Pro-Val-Pro-Ile-Thr-Glu-Tyr-Ser-Thr-Gly-Gly-Arg-Gly-Glu-Leu-Val-Pro- (SEQ ID No. 8)
   It was not possible to discriminate whether Asx3 was an Asp or Asn residue. Xaa1 was unidentified.
*Peptide 7:*
   Gly-Ala-Leu-Asx-Asp-Ile-Val-Asn-Gly-Thr-Ser-Val-Gly-Gln-Asp-Gly-Arg-Asn-Arg-Phe-Gly-Gly-Thr-Pro-Asn-Gly-Ser- (SEQ ID No. 9)
   It was not possible to discriminate whether Asx4 was an Asp or Asn residue. Note that Asn25 is not glycosylated although it is found in the concensus sequence for N-glycosylation.

### Peptide sequences of A. oryzae TPAP

N-terminal amino acid sequencing intact TPAP as well as of peptides derived from TPAP was carried out in an Applied Biosystems 473A protein sequencer according to the manufacturers instructions.

The N-terminal amino acid sequence of intact TPAP were determined following SDS-PAGE and electroblotting onto a PVDF-membrane using standard procedures. The following 23 residue amino acid sequence was found:
Ala-Lys-Xaa-Ile-Ser-His-Yaa-Asp-Ser-Ile-Ile-Thr-Pro-Pro-Yaa-Leu-Lys-Glu-Leu-Tyr-Asn-Ile-Gly- (SEQ ID NO 14)

This sequence is clearly homologous to the N-terminal amino acid sequence of TPAP from *A. niger*. Based on this homology Xaa most likely is a glycosylated Asn-residue while Yaa probably represents Cys-residues.

Following denaturation, reduction and S-carboxymethylation, peptides were derived from TPAP by proteolytic cleavage using the lysyl-specific protease from *Achromobacter.* The resulting peptides were fractionated and repurified using revesed phase HPLC. The purity and mass of the peptides were evaluated using matrix assisted laser desorption ionisation time-of-flight mass spectrometry using a VG Analytical TofSpec operated according to the manufacturers recommandations. The following 4 peptides were sequenced.
*Peptide 8:*
   Glu-Leu-Tyr-Asn-Ile-Gly-Asp-Tyr-Gln-Ala-Asp-Ala-Asn-Ser-Gly-Ser-Lys (SEQ ID NO 10)
   This peptide overlaps with the 6 last amino acid residues determined by the N-terminal sequencing of intact TPAP thereby extending the N-terminal sequence to 34 residues.
*Peptide 9:*
   Thr-Thr-Pro-Glu-Arg-Gly-Thr-Tyr-Phe-Ser-Ser-Gly-Gly-Phe-Ser-Asn-Tyr-Trp-Pro-Arg-Pro-Glu-Trp-Gln-Asn-Gln-Ala-Val-Ala-Ser-Tyr-Leu- (SEQ ID NO 11)
   This peptide is homologous to peptide 3 from *A*. *niger* TPAP.
*Peptide 10 :*
   Gly-Thr-Leu-Gly-Glu-Phe-Asp-Gly-Thr-Ser-Ala-Ser-Ala-Pro-Ala-Phe-Ser-Ala-Val-Ile-Ala-Leu-Leu-Asn-Asp-Ala-Arg-Leu-Arg-Ala-Gly-Lys-Pro-Thr-Leu-Gly-Phe-Leu-Asn-Pro-Trp-Leu-Tyr-Lys (SEQ ID NO 12)
*Peptide 11:*
   Thr-Gly-Arg-Gln-Gly-Leu-Gln-Asn-Ile-Thr-Leu-Gly-Ala-Ser-Ile-Gly-Xaa-Thr-Gly-Arg-Ala-Arg-Phe-Gly-Gly-Ala-Pro-Asn-Gly-Gly-Pro-Val-Val-Pro-Tyr-Ala-Ser- (SEQ ID NO 13),

Xaa designates an unidentified residue.

### EXAMPLE 4

**The amino acid compositions** of *A*. *niger* TPAP forms I and II were determined. Duplicates of lyophilyzed aliqouts of TPAP forms I and II were hydrolysed in 6 N HCl containing 0.1% phenol at 110°C in vacuo for 16 h. Trypthophan was determined following hydrolysis in 3M methanesulfonic acid. Following hydrolysis the amino acid compositions were determined using an Applied Biosystems 420A amino acid analysis system operated according to the manufacturers instructions. The results show that within experimental error TPAP forms I and II have identical amino acid compositions as shown in Table 4 below.

**Table 4**

| Amino acid composition of *A*. *niger* TPAP forms I and II. | | |
|---|---|---|
| | TPAP I | TPAP II |
| | (Mol%) | (Mol%) |
| Asx | 13.1 | 13.4 |
| Glx | 8.2 | 7.9 |
| Ser | 10.5 | 10.7 |
| Gly | 13.7 | 13.1 |
| His | 0.5 | 0.5 |
| Arg | 3.1 | 3.1 |
| Thr | 5.7 | 5.6 |
| Ala | 8.0 | 7.9 |
| Pro | 7.2 | 7.3 |
| Tyr | 3.7 | 3.8 |
| Val | 6.0 | 5.9 |
| Met | 0.3 | 0.5 |
| Cys | 0.6 | 0.7 |
| Ile | 2.5 | 2.5 |
| Leu | 8.4 | 8.4 |
| Phe | 4.5 | 4.8 |
| Lys | 3.1 | 3.0 |
| Trp | 0.9 | 1.0 |

### EXAMPLE 5

**The monosaccharide compositions** of *A*. *niger* TPAP forms I and II were determined. Duplicates of lyophilyzed aliqouts of TPAP forms I and II were hydrolysed in 2 M TFA at 100°C in vacuo for 1 h, 2 h and 4 h. Following hydrolysis the monosaccharide compositions were analysed by high performance ion exchange chromatography using a CarboPac^{™} PA1 column eluted with 16 mM NaOH. The monosaccharides were detected by pulsed amperometric detection. Due to the different stability and release of the monosaccharides in 2 M TFA the amount of galactose was determined after 1 h hydrolysis, the amount of mannose after 2 h hydrolysis and the amount of glucosamine after 4 h hydrolysis. The results obtained indicate very minor differences in mannose content of TPAP form I and II as shown in the table below.

**Table 5**

| Monosaccharide compositions of TPAP forms I and II. The results are given in pmol monosaccharide/pmol protein as determined from amino acid analysis. | | |
|---|---|---|
| | TPAP form I | TPAP form II |
| | (pmol/pmol) | (pmol/pmol) |
| Glucosamine | 4 | 4 |
| Galactose | 12 | 11 |
| Mannose | 52 | 47 |

### EXAMPLE 6

### CLEAVAGE BY A. niger TPAP

The ability of *A*. *niger* TPAP to destabilize the AMG G1 form was investigated using purified AMG and TPAP (obtained as described in the Materials and Methods section in Example 1) at TPAP/AMG ratios equal to those in formulated products. Amino acid sequencing of the destabilized preparations revealed a modification in the N-terminal of the catalytic domain of AMG. A tripeptide comprising the first three amino acid residues (Ala-Thr-Leu) had been cleaved off by the peptidase indicating that the enzyme is a tripeptidyl aminopeptidase. The classification as a tripeptidyl *amino*peptidase, based on the cleavage of AMG as well as of the chromogenic substrate Phe-Pro-Ala-pNA, was further supported by the lack of activity towards another chromogenic substrate Succinyl-Ala-Ala-Ala-pNA. In this substrate the free amino group at the N-terminus has been succinylated and thereby made the substrate inaccesible to cleavage by TPAP. The TPAP cleavage of the AMG batches was not complete after 3 weeks storage, as a mixture of intact and truncated AMG (AMG_{trunc}) was detected by amino acid sequencing.

The destabilization of AMG G1 obtained with various dosages of TPAP at different temperatures has been investigated. A TPAP dosage in the range from 3 to 10 µg/ml gave a significant destabilization of AMG and also TPAP/AMG ratios similar to those measured in several fermentations. The destabilization effect was most pronounced at 37°C but also at 25°C a clear effect on the thermostability was observed after 27 days of storage. Storage at 4°C did not affect the thermostability of AMG.

### EXAMPLE 7

**The specificity of *A. niger* TPAP** was investigated using various native protein and peptide substrates. Using these substrates it was found that TPAP is highly unspecific with respect to the amino acid residue N-terminal to the cleavage point. TPAP is capable of cleavage even following Pro residues and CM-Cys residues. However, a Pro residue C-terminal to the cleavage point completely inhibits cleavage by TPAP. Specific cleavage products obtained from subjecting native proteins to TPAP treatment include the tripeptide sequences IPE, YVD, WRQ, KGA, LPS, ANL, NGT, LMQ, YFE, GPG, GGG, ADG, RST, SVE, KKP, EGV, NTG, AGD, RHN, LKT, VEK, KPE, GVN, TGA, GDR, HNL, HSQ, GTF, TSD, YSK, YLD, SRR, AQD, FVQ, WLM and ATL.

### EXAMPLE 8

### INHIBITION OF A. niger TPAP ACTIVITY

Inhibition of TPAP by the protease inhibitor, cloromethylketone Ala-Ala-Phe-CMK (AAF-CMK), was tested under the conditions described above. AAF-CMK was found to inhibit TPAP completely.

Addition of AAF-CMK to fermentation broths can totally inhibit the TPAP activity. The thermostability of 5 different AMG batches were investigated with and without the TPAP activity present. The thermostability of all 5 AMG batches were unchanged after 2 weeks storage at 37 °C when TPAP had been inhibited. The corresponding samples without inhibitor were all clearly destabilized.

### EXAMPLE 9

Portions of 10 ml culture broths obtained from a cultivation of an AMG producing *A*. *niger* strain were adjusted to either pH 6.5 or pH 7.0 with sodium hydroxide. The samples were incubated at 25°C, 40°C and 50°C, respectively, for one hour and subsequently the samples were adjusted to pH 4.3 with acetic acid. The storage stability of the treated samples was measured. It was shown that this simple recovery process (based on a pH treatment at elevated temperatures) resulted in efficient removal of TPAP activity (Table 6). Treatment of the culture broth at pH 6.5/40°C for one hour reduced the TPAP activity to 5% and gave a very stable product with an unchanged thermostability after two weeks of storage at 40°C.

**Table 6**

| pH/temp. | TPAP after treatment | AMG after treatment | T₃₀ | T₃₀ | TPAP |
|---|---|---|---|---|---|
| 1 hour | | | initial | 2 weeks | 2 weeks |
| Reference | 100 | 100 | 50 | 31 | 80 |
| 6.5/25 C | 90 | 100 | 46 | 43 | 42 |
| 6.5/40c | 5 | 97 | 47 | 53 | <1 |
| 6.5/50c | <1 | 90 | - | - | - |
| 7.0/25 c | 86 | 99 | 48 | 44 | <1 |
| 7.0/40 c | <1 | 92 | 48 | 55 | <1 |
| 7.0/50 c | <1 | 85 | - | - | - |

All numbers in the table are %. The values indicated for TPAP and AMG, respectively, are the relative activity at the time indicated.

### EXAMPLE 10

### PCR CLONING

From the N-terminal amino acid sequence of *A. niger* TPAP the sequence of which is shown in SEQ ID No. 3 four PCR primers were designed (Table 7) Genomic DNA from a strain of A. niger was used as template in four PCR reactions. PCR fragments in the expected size 65 bp was purified and cloned into the plasmid pCR^{™} II (Invitrogen Corporation). Sequencing of the insert for three clones showed that two of them contains the degenerate sequence in the areas corresponding to the primers, while the sequence in between was invariant to the N-terminal amino acid sequence.

In order to clone a larger DNA fragment encoding the tripeptidyl aminopeptidase the primer #6010 (GCACTGTCGAAGCAGCTGTACAACATCGGTG) corresponding to the invariant sequence was made. From two other peptide sequences three PCR primers were designed (Table 7). Three PCR reactions (#6010/#5988, #6010/#5989, and #6010/#5990 were performed using genomic *A. niger* DNA as template. The reactions were done at two annealing temperatures 42°C and 45°C. Reactions #6010/#5989 (one fragment or approx. 80 bp) and #6010/#5990 (three fragments of approx. 120 bp, 500 bp, and 950 bp) show the same band pattern on an agarose gel at the two different temperatures. In reaction #6010/#5988 a fragment of approx 120 bp was seen at 42°C, but at 45°C a fragment of approx. 950 bp was seen. The 950 bp fragment turned out to be the right one and was inserted into the pCR II AT vector. An *E. coli* strain harbouring the 950 kb fragment was deposited with the Deutsche Sammlung von Microorganismen und Zellkulturen GmbH, Mascheroder Weg 1b, D-38124 Braunschweig, Germany on December 5, 1994, as DSM 9570, under the provisions of the Budapest Treaty.

**Table 7**

| Oligo Nucleotide Primers | |
|---|---|
| **#5765** | |
| 5'- G A C T C C A T C A T C A C C C C T     T     T     T     T A     A     A     A G                 G | |
| | |
| **#5766** | |
| 5'- G A C A G C A T C A T C A C C C C T     T     T     T     T A G | |
| | |
| **#5767** | |
| | C T G A T G G T T C G G C T G G G - 5' A       A       C       A       A |
| | |
| **#5768** | |
| | C T G A T G G T T C G C C T G G G - 5' A     A       C     T     A |
| | |
| **#5988** | |
| | A T A C G A C A A A T A C T A T T - 5' G     C     C     G     G G     G T     T |
| **#5989** | |
| | A T A A A A C T C C T C A T A C G - 5' G G   C     T     T     G G |
| | |
| **#5990** | C T T C T A A A A C T T G T T G T - 5' C       G      G      C     C |

The 950 bp fragment was sequenced from both ends. It was found that this fragment encodes the N-terminal part of TPAP. The partial sequence of the N-terminal part is shown in SEQ ID No. 2, the partial sequence obtained from sequencing from the other end is shown in SEQ ID No. 3. The sequence of the entire "950 base fragment" which was found to be constituted by 908 bp, is shown in SEQ ID No. 1.

### Southern analysis

Genomic DNA from a strain of *A.niger* and from *A*. *oryzae* IFO 4177 was isolated as described previously (Yelton et al., 1984, Proc. Natl. Acad. Sci. USA. 81: 1470-1474). Genomic DNA was digested with appropriate restriction enzymes, fractionated on a 0.7% agarose gel, and blotted to Immobilon^{™}-N as described by the manofacturer. The membranes were probed for the presence of the 950 bp TPAP gene sequence labelled with alpha-32^{P} dATP (NewEngland) by random priming according to the method described by Feinberg et al., 1983, Anal. Biochem. 132:6. The membranes were then incubated for 2 hours at 65°C in hybridization solution (5xSSC (0.15 M NaCl, 0.015 M trisodium citrate), 10xDenhardt (0.2% Ficoll, 0.2% polyvinyl pyrrolidone, 0.2% bovine serum albumin), 10 mM EDTA, 1% SDS, 150µg/ml Poly A, 50 µg/ml yeast RNA). Next, the radioactively labelled probe was added and incubated at 65°C overnight with gentle agitation. The memebranes were washed twice at 30°C for 15 minutes in 2XSSC, 1% SDS. Finally, the membranes were dried, covered with plastic wrap, and exposed to X-ray film (Fuli-RX) at -70°C.

The results show that both strains contain only one gene encoding the TPAP.

### SEQUENCE LISTING

SEQ ID No. 1
   908 bases. 
CACTGTCTGAAGCAGCTGTACAACATCGGTGACTACCAGGCCGATCCCAAGTCCGGCAG-
CAAGATCGGCTTTGCCAGCTACCTTGAGGAATACGCCCGGTATGCCGATCTCGAGAGG-
TTCGAGCAGCACCTGGC TCCCAATGCCATCGGCCAGAACTTCAGCGTCGTCCAATTCAACG-
GCGGCCTCAACGATCAGCTTTCA TCGAGTGACAGCGGCGAAGCCAACCTCGACCTGCAGTA-
CATCCTGGGCGTCAGCGCTCCCGTCCCCA TCACCGAGTACAGCACCGGCGGACGCGGCGAA-
CTAGTCCCCGACCTGAGCTCCCCCGACCCCAACGA CAACAGCAACGAGCCCTACCTTGACT-
TCCTTCAGGGAATCCTCAAGCTTAACAACTCCGACCTCCCA CAAGTCATCTCTACCTCCTA-
CGGTGAAGACGAACAGGTATGCACCTCACCTGACCCATTCCATTTA CATCCTCACCTCT-
CTCAACCAAACTAACAACACCAACAGACTATCCCCGTCCCCTACGCCCGCACC GTCTGCAA-
CCTCTACGCCCAACTCGGCAGCCGCGGCGTCTCTGTAATCTTCTCCAGCGGCGACTCCG
GCGTCGGCGCCGCCTGCCTCACCAACGACGGCACCAACCGCACGCACTTCCCTCCTCAATT
CCCCGC CTCCTGCCCCTGGGTAACCTCCGTCGGCGCAACCTCCAAGACCTCCCCCGAGCAA-
GCCGTCTCCTTC TCCTCCGGCGGCTTCTCCGACCTCTGGCCCGCCCCTCCTACCAACACG-
CCGCCGTGCAAACCTACC TCACCAAGCACCTGGGCAACAAGTTCTCGGGGCTTTTCAACGC-
CTCCGGCCGCGCCTTCCCCGACGT CTCCGCGCAGGGCGTCAACTACGCTGTTTACGACAAA
SEQ ID No. 2 
CACTGTCTGAAGCAGCTGTACAACATCGGTGACTACCAGGCCGATCCCAAGTCCGGCAGCA
AGATCGGCTTGGGCAGCTACCTTGAGGAATACGCCCGGTATGCCGATCTCGAGAGG
TTCGAGCAGCACCTGGCTCCAATGCATCGGCAGAACTCAGCGTCGTCCAATTCACGGCGGCT-
CACGATCAGCTTCATCGAGTGACAGCGGCGAGCAACTCGACTGCAGTAC
SEQ ID No. 3 
CCTCCTACCAACACGCCGCCGTGCAACCTACCTGACCAAGCACCTGGCAACAAGTTCTCGG-
GGCTTTTCAACGCCTCCGGCCGCGCCTTCCCCGACGTCTCCGCGCAGGGCGTCAACTACGCT-
GTTTACGACAA
SEQ ID No. 4 
Ala-Gln-Asn-Thr-Ser-His-Cys-Asp-Ser-Ile-Ile-Thr-Pro-His-Cys-
Leu-Lys-Gln-Leu-Tyr-Asn-Ile-Gly-Asp-Tyr-Gln-Ala-Asp-Pro-Lys
SEQ ID No. 5 
Thr-Ser-Pro-Glu-Gln-Ala-Val-Ser-Phe-Ser-Ser-Gly-Gly-Phe-Ser-
Asp-Leu-Trp-Pro-Arg-Pro-Ser-Tyr-Gln-His-
SEQ ID No. 6 
Phe-Ser-Gly-Leu-Phe-Asn-Ala-Ser-Gly-Arg-Ala-Phe-Pro-Asp-Val-
Ser-Ala-Gln-Gly-Val-Asn-Tyr-Ala-Val-Tyr-Asp-Lys
SEQ ID No. 7 
Ile-Gly-Phe-Ala-Ser-Tyr-Leu-Gln-Glu-Tyr-Ala-Arg-Tyr-Ala-Asx-
Leu-Glu-Arg-Phe-Glu-Gln-His-Leu-
SEQ ID No. 8 
Xaa-Leu-Asx-Leu-Gln-Tyr-Ile-Leu-Gly-Val-Ser-Ala-Pro-Val-Pro-
Ile-Thr-Glu-Tyr-Ser-Thr-Gly-Gly-Arg-Gly-Glu-Leu-Val-Pro-
SEQ ID No. 9 
Gly-Ala-Leu-Asx-Asp-Ile-Val-Asn-Gly-Thr-Ser-Val-Gly-Gln-Asp-
Gly-Arg-Asn-Arg-Phe-Gly-Gly-Thr-Pro-Asn-Gly-Ser-
SEQ ID NO. 10 
Glu-Leu-Tyr-Asn-Ile-Gly-Asp-Tyr-Gln-Ala-Asp-Ala-Asn-Ser-Gly-
Ser-Lys
SEQ ID NO. 11 
Thr-Thr-Pro-Glu-Arg-Gly-Thr-Tyr-Phe-Ser-Ser-Gly-Gly-Phe-Ser-
Asn-Tyr-Trp-Pro-Arg-Pro-Glu-Trp-Gln-Asn-Gln-Ala-Val-Ala-Ser-
Tyr-Leu-
SEQ ID NO. 12 
Gly-Thr-Leu-Gly-Glu-Phe-Asp-Gly-Thr-Ser-Ala-Ser-Ala-Pro-Ala-
Phe-Ser-Ala-Val-Ile-Ala-Leu-Leu-Asn-Asp-Ala-Arg-Leu-Arg-Ala-
Gly-Lys-Pro-Thr-Leu-Gly-Phe-Leu-Asn-Pro-Trp-Leu-Tyr-Lys
SEQ ID NO. 13 
Thr-Gly-Arg-Gln-Gly-Leu-Gln-Asn-Ile-Thr-Leu-Gly-Ala-Ser-Ile-
Gly-Xaa-Thr-Gly-Arg-Ala-Arg-Phe-Gly-Gly-Ala-Pro-Asn-Gly-Gly-
Pro-Val-Val-Pro-Tyr-Ala-Ser-
SEQ ID NO 14 
Ala-Lys-Xaa-Ile-Ser-His-Yaa-Asp-Ser-Ile-Ile-Thr-Pro-Pro-Yaa-
Leu-Lys-Glu-Leu-Tyr-Asn-Ile-Gly-
SEQ ID NO 15

Ala-Xaa(1)-Asn-Xaa(2)-Ser-His-Cys-Asp-Ser-Ile-Ile-Thr-Pro-Xaa
(3)-Cys-Leu-Lys-Xaa(4)-Leu-Tyr-Asn-Ile-Gly-Asp-Tyr-Gln-Ala-Asp-
Xaa(5)-Xaa(6)

## Claims

1. A method of reducing or eliminating the tripeptidyl aminopeptidase (TPAP) production from a TPAP producing cell of the genus *Aspergillus,* in which method a DNA sequence encoding TPAP or a part thereof essential for exhibiting TPAP activity or the TPAP promoter sequence required for the expression of TPAP from a TPAP encoding DNA sequence present in said cell are modified or inactivated so as to result in a reduced or no TPAP production from said cell.

2. The method according to claim 1, in which the modification or inactivation of the DNA sequence is obtained by subjecting the TPAP producing cell to mutagenesis and selecting for cells for which the TPAP producing capability has been reduced or removed.

3. The method according to claim 2, in which the mutagenesis is performed by random or site-directed mutagenesis.

4. A method of reducing or eliminating the TPAP production from a TPAP producing cell of the genus *Aspergillus,* in which method a nucleotide sequence complementary to the TPAP encoding sequence and thus capable of hybridizing to TPAP mRNA produced in the cell is introduced into the cell in such a manner that the nucleotide sequence may be transcribed in the cell under conditions allowing the nucleotide sequence to hybridize to the TPAP mRNA and thus reduce the amount of TPAP translated from said mRNA or eliminate any such translation.

5. A host cell prepared according to claims 1-4.

6. A method of preparing a product wherein at least 60% of the TPAP activity is removed, which method comprises transforming a host cell according to claim 5 with a DNA sequence encoding the product, culturing the transformed cell under suitable conditions for expression of the product, and recovering the product from the culture.

7. A method of preparing a product wherein at least 60% of the TPAP activity is removed, which product is encoded by a DNA sequence present in a TPAP expressing cell, which method comprises modifying or inactivation a DNA sequence present in said cell and encoding TPAP or a part thereof essential for exhibiting TPAP activity or the TPAP promoter required for the expression of TPAP from a TPAP encoding DNA sequence according to the method of any of claims 1-4 and subsequently culturing the cell under suitable conditions for expression of the product, and recovering the product from the culture.

8. A method of preparing a product wherein at least 60% of the TPAP activity is removed by fermentation of a TPAP-producing cell of the genus *Aspergillus* which also produces the product, which method comprises adding an effective amount of Ala-Ala-Phe-chloromethylketone to the fermentation broth either during or after the fermentation has been completed, recovering the product of interest from the fermentation broth, and optionally subjecting the recovered product to further purification.

9. A method of preparing a product wherein at least 60% of the TPAP activity is removed by fermentation of a TPAP-producing cell of the genus *Aspergillus* which also produces the product, which method comprises subjecting the fermentation broth or an enzyme preparation isolated there from containing TPAP and the product of interest to a combined pH and temperature treatment for a sufficient period of time to reduce the TPAP activity, and optionally recovering the product from the treated broth.

10. The method according to claim 9, in which the TPAP reducing treatment is performed at a pH in the range of 6.5-7 and at a temperature in the range of 25-40° C.

11. The method according to any of claims 6-10, in which the product is an enzyme.

12. The method according to claim 11, in which the product is selected from an amylolytic enzyme, a lipolytic enzyme, a proteolytic enzyme, a cellulytic enzyme, an oxidoreductase, and a plant cell-wall degrading enzyme.

13. The method according to claim 12, in which the product is AMG, lipase, cutinase, cellulase, amylase, protease, peroxidase, transglutaminase, laccase, catalase, glucose oxidase, or phytase.

## Patentansprüche

1. Verfahren zur Reduzierung oder Eliminierung der Tripeptidyl-Aminopeptidase-(TPAP)-Erzeugung aus einer TPAP-produzierenden Zelle der Gattung *Aspergillus,* wobei in dem Verfahren eine in besagter Zelle vorhandene DNA-Sequenz, die TPAP oder ein für das Aufweisen der TPAP-Aktivität essentiellen Teil davon oder die TPAP-Promotorsequenz kodiert, die für die Expression von TPAP von einer TPAP-kodierenden DNA-Sequenz benötigt wird, modifiziert oder inaktiviert wird, so dass dies in einer reduzierten oder in keiner TPAP-Erzeugung in besagter Zelle resultiert.

2. Verfahren nach Anspruch 1, bei dem die Modifizierung oder Inaktivierung der DNA-Sequenz erzielt wird, indem die TPAP-produzierende Zelle einer Mutagenese und einer Selektion auf Zellen unterzogen wird, in denen die TPAP-produzierende Fähigkeit reduziert oder entfernt wurde.

3. Verfahren nach Anspruch 2, bei dem die Mutagenese durch zufallsbedingte oder stellenspezifische Mutagenese durchgeführt wird.

4. Verfahren zur Reduzierung oder Eliminierung der TPAP-Erzeugung aus einer TPAP-produzierenden Zelle der Gattung *Aspergillus,* wobei in dem Verfahren eine Nukleotidsequenz, komplementär zur TPAP-kodierenden Sequenz und somit in der Lage, mit in der Zelle produzierten TPAP mRNA zu hybridisieren, in die Zelle in solch einer Art und Weise eingefügt wird, dass die Nukleotidsequenz in der Zelle unter Bedingungen transkribiert werden kann, die es der Nukleotidsequenz erlauben, mit TPAP mRNA zu hybridisieren und somit die Menge des translatierten TPAP von besagter mRNA zu reduzieren oder jedwede Translation zu eliminieren.

5. Wirtzelle hergestellt gemäß den Ansprüchen 1-4.

6. Verfahren zur Herstellung eines Erzeugnisses, worin mindestens 60 % der TPAP-Aktivität entfernt sind, wobei das Verfahren das Transformieren einer Wirtszelle gemäß Anspruch 5 mit einer das Erzeugnis kodierenden DNA-Sequenz, das Kultivieren der transformierten Zelle unter zur Expression des Erzeugnisses geeigneten Bedingungen, und das Wiedergewinnen des Erzeugnisses aus der Kultur umfasst.

7. Verfahren zur Herstellung eines Erzeugnisses, worin mindestens 60 % der TPAP-Aktivität entfernt sind, wobei das Erzeugnis durch eine in der TPAP-exprimierenden Zelle vorhandene DNA-Sequenz kodiert wird, wobei das Verfahren die folgenden Schritte umfasst: das Modifizieren oder Inaktivieren einer DNA-Sequenz, die in besagter Zelle vorhandenen ist und TPAP oder ein für das Aufweisen der TPAP-Aktivität essentiellen Teil davon oder den TPAP-Promotor kodiert, der für die Expression von TPAP von einer TPAP-kodierenden DNA-Sequenz benötigt wird, gemäß dem Verfahren nach einem der Ansprüche 1-4, und das anschließende Kultivieren der Zelle unter zur Expression des Erzeugnisses geeigneten Bedingungen und das Wiedergewinnen des Erzeugnisses aus der Kultur.

8. Verfahren zur Herstellung eines Erzeugnisses, worin mindestens 60 % der TPAP-Aktivität durch Fermentierung einer TPAP-produzierenden Zelle der Gattung *Aspergillus,* welche ebenfalls das Erzeugnis herstellt, entfernt sind, wobei das Verfahren die Zugabe einer wirksamen Menge von Ala-Ala-Phe-Chlormethylketon, entweder während oder nachdem die Fermentierung beendet wurde, zum Fermentationsmedium, das Wiedergewinnen des gewünschten Erzeugnisses aus dem Fermentationsmedium, und wahlweise das Unterziehen des wiedergewonnenen Erzeugnisses einer weiteren Aufreinigung umfasst.

9. Verfahren zur Herstellung eines Erzeugnisses, worin mindestens 60 % der TPAP-Aktivität durch Fermentierung einer TPAP-produzierenden Zelle der Gattung *Aspergillus,* welche ebenfalls das Erzeugnis herstellt, entfernt sind, wobei das Verfahren die folgenden Schritte umfasst: das Unterziehen des Fermentationsmediums oder einer daraus isolierten, TPAP und das gewünschte Erzeugnis enthaltenen Enzympräparation einer kombinierten pH- und Temperatur-Behandlung für eine zur Reduktion der TPAP-Aktivität ausreichende Zeitdauer, und wahlweise das Wiedergewinnen des Erzeugnisses aus dem behandelten Medium.

10. Verfahren nach Anspruch 9, bei dem die TPAP-reduzierende Behandlung bei einem pH im Bereich von 6.5 - 7 und bei einer Temperatur im Bereich von 25 - 40 ° C durchgeführt wird.

11. Verfahren nach einem der Ansprüche 6-10, bei dem das Erzeugnis ein Enzym ist.

12. Verfahren nach Anspruch 11, bei dem das Erzeugnis ausgewählt wird aus der Gruppe bestehend aus einem amylolytischen Enzym, einem lipolytischem Enzym, einem proteolytischem Enzym, einem zellulytischem Enzym, einer Oxidoreduktase, und einem Pflanzenzellwand-abbauendem Enzym.

13. Verfahren nach Anspruch 12, bei dem das Erzeugnis eine AMG, Lipase, Kutinase, Zellulase, Amylase, Protease, Peroxidase, Transglutaminase, Laccase, Katalase, Glukose-Oxidase oder Phytase ist.

## Revendications

1. Méthode pour diminuer ou éliminer la production de tripeptidyl aminopeptidase (TPAP) par une cellule produisant de la TPAP du genre *Aspergillus.* Méthode dans laquelle une séquence d'ADN codant pour la TPAP ou pour une partie de celle-ci essentielle pour montrer une activité TPAP, ou la séquence promotrice de TPAP nécessaire pour l'expression de TPAP par une séquence d'ADN codant pour la TPAP présente dans ladite cellule sont modifiées ou inactivées de manière à obtenir une production réduite ou aucune production de TPAP par ladite cellule.

2. Méthode selon la revendication 1, dans laquelle la modification ou inactivation de la séquence d'ADN est obtenue en soumettant la cellule produisant de la TPAP à une mutagenèse et en sélectionnant les cellules dans lesquelles la capacité de production de la TPAP a été réduite ou perdue.

3. Méthode selon la revendication 2, dans laquelle la mutagenèse est faite par mutagenèse aléatoire ou dirigée.

4. Méthode pour diminuer ou éliminer la production de TPAP par une cellule produisant de la TPAP du genre *Aspergillus.* Méthode dans laquelle une séquence nucléotidique complémentaire de la séquence codant pour la TPAP et donc capable de s'hybrider à un ARNm de la TPAP produit dans la cellule est introduite dans la cellule de manière à ce que la séquence nucléotidique puisse être transcrite dans la cellule dans des conditions permettant à la séquence nucléotidique de s'hybrider à l'ARNm de la TPAP et ainsi de réduire la quantité de TPAP traduite à partir dudit ARNm ou d'éliminer une telle traduction.

5. Hôte cellulaire préparé selon l'une des revendications 1 à 4.

6. Méthode pour préparer un produit dans lequel au moins 60% de l'activité TPAP a été retirée, la méthode comprenant la transformation d'une cellule hôte selon la revendication 5 avec une séquence d'ADN codant le produit, la culture de la cellule transformée dans des conditions adaptées à l'expression du produit, et la récupération du produit à partir de la culture.

7. Méthode pour préparer un produit dans lequel au moins 60 % de l'activité TPAP a été retirée, ledit produit étant codé par une séquence d'ADN présente dans la cellule exprimant la TPAP, la méthode comprenant la modification ou l'inactivation d'une séquence d'ADN présente dans ladite cellule et codant pour la TPAP ou pour une partie de celle-ci essentielle pour montrer une activité TPAP ou de la séquence promotrice de TPAP nécessaire pour l'expression de TPAP par une séquence d'ADN codant pour la TPAP selon la méthode d'une quelconque des revendications 1-4, et ensuite la culture de la cellule dans des conditions adaptées à l'expression du produit, et la récupération du produit à partir de la culture.

8. Méthode pour préparer un produit dans lequel au moins 60 % de l'activité TPAP a été retirée par fermentation d'une cellule produisant la TPAP du genre *Aspergillus* qui produit également le produit, la méthode comprenant l'ajout d'une quantité efficace de Ala-Ala-Phe-chlorométhylcétone au moût de fermentation soit pendant soit après que la fermentation ait été accomplie, la récupération du produit d'intérêt à partir du moût de fermentation, et facultativement la soumission du produit récupéré à une purification supplémentaire.

9. Méthode pour préparer un produit dans lequel au moins 60 % de l'activité TPAP a été retirée par fermentation d'une cellule produisant la TPAP du genre *Aspergillus* qui produit également le produit, la méthode comprenant la soumission du moût de fermentation ou d'une préparation d'enzymes isolée de celui-ci et contenant la TPAP et le produit d'intérêt à un traitement combiné de pH et de température pendant une période de temps suffisante pour réduire l'activité TPAP et facultativement la récupération du produit à partir du moût traité.

10. Méthode selon la revendication 9, dans laquelle le traitement réduisant la TPAP est fait à un pH dans la gamme de 6,5-7 et à une température dans la gamme de 25-40°C.

11. Méthode selon l'une quelconque des revendications 6-10, dans laquelle le produit est une enzyme.

12. Méthode selon la revendication 11 dans laquelle le produit est sélectionné parmi une enzyme amylolytique, une enzyme lipolytique, une enzyme protéolytique, une enzyme cellulytique, une oxydoréductase, et une enzyme dégradant la paroi cellulaire de plante.

13. Méthode selon la revendication 12, dans laquelle le produit est une AMG, une lipase, une cutinase, une cellulase, une amylase, une protéase, une peroxydase, une transglutaminase, une laccase, une catalase, une glucose oxydase, ou une phytase.
